(19) Europäisches Patentamt
European Patent Office
Office européen des brevets

(11) EP 1 080 728 A1

(12) **EUROPEAN PATENT APPLICATION**
published in accordance with Art. 158(3) EPC

(43) Date of publication:
**07.03.2001 Bulletin 2001/10**

(21) Application number: **00909634.8**

(22) Date of filing: **15.03.2000**

(51) Int. Cl.[7]: **A61K 45/00**, A61K 31/557, A61P 43/00, A61P 13/12

(86) International application number:
**PCT/JP00/01556**

(87) International publication number:
**WO 00/54808 (21.09.2000 Gazette 2000/38)**

(84) Designated Contracting States:
**AT BE CH CY DE DK ES FI FR GB GR IE IT LI LU MC NL PT SE**
Designated Extension States:
**AL LT LV MK RO SI**

(30) Priority: **16.03.1999 JP 6969699**

(71) Applicant:
**TORAY INDUSTRIES, INC.**
**Tokyo 103-8666 (JP)**

(72) Inventors:
- **KUMAGAI, Hiroki**
  **Kamakura-shi, Kanagawa 248-0034 (JP)**
- **OCHI, Yasuo**
  **Kamakura-shi, Kanagawa 248-0036 (JP)**
- **HAYASHI, Ryoji**
  **Fujisawa-shi Kanagawa 251-0033 (JP)**

(74) Representative: **Kador & Partner**
**Corneliusstrasse 15**
**80469 München (DE)**

(54) **PROSTAGLANDIN EP4 RECEPTOR AGONIST AND TREATMENT METHOD**

(57) The present invention relates to a prostaglandin EP4 receptor ligand comprising a prostaglandin $I_2$ derivative as an effective ingredient. The compound according to the present invention is useful as a pharmaceutical tool for making physiological actions related to prostaglandin EP4 receptor clear, or as a medicine for use in prevention/treatment of prostaglandin EP4 receptor-mediated diseases.

EP 1 080 728 A1

## Description

### Technical Field

The present invention relates to a prostaglandin EP 4 receptor ligand, and a new application of prostaglandin $I_2$ derivatives.

### Background Art

Prostaglandin $E_2$ (abbreviated as $PGE_2$ hereinafter), which is produced as metabolites of arachidinic acid in vivo, has a wide range of physiological actions such as uterine constrictive action, suppressive action of gastric acid secretion, protective action of the gastric mucous membrane, stimulative action of digestive peristalsis, fever and diarrhea inducing action, or the like. It has been revealed from advances of recent studies that these physiological actions of $PGE_2$ are expressed by binding to specific $PGE_2$ receptors in vivo. The receptors for binding of $PGE_2$ can be classified into four receptor subtypes, which are named as the EP1, EP2, EP3 and EP4 receptor [Coleman, R. A. et al., Pharmacol. Rev., 46, 205-229 (1994)]. It has been also made clear that these receptor subtypes are related to different physiological functions respectively.

The EP4 receptor among these receptors is distributed in such organs as the heart, kidney, liver, intestine, lung and bone. It has been known that the receptor is related to relaxation of the smooth muscles, differentiation and proliferation of lymphocytes, proliferation of mesangial cells, and collagen production of the fibroblasts, or the like. The compounds that bind to EP4 receptor have a possibility to serve as preventives or remedies (for example against nephrosclerosis).

Examples of the compounds that bind to EP4 receptor, that is to say EP4 receptor ligands, include prostanoic acid derivatives such as $PGE_2$, 3,7-dithiaprostanoic acid disclosed in Japanese Unexamined Patent Application Publication No. 10-265454, and oxazole derivatives disclosed in International Unexamined Patent Application Publication No. 9855468.

The object of the present invention is to provide a prostaglandin EP4 receptor ligand having a novel molecular frame.

### Disclosure of Invention

The present invention provides a prostaglandin EP4 receptor ligand comprising prostaglandin $I_2$ derivatives as effective ingredients, and a method for treating prostaglandin EP4 receptor-mediated diseases.

### Best Mode for Carrying Out the Invention

While the prostaglandin $I_2$ derivatives according to the present invention include methaphenylene, carbacyclin and isocarbacyclin type prostaglandin $I_2$ derivatives, they are not particularly specified. Preferably, a methaphenylene type prostaglandin $I_2$ derivative is used, and the particularly preferable methaphenylene type prostaglandin $I_2$ derivative is 4,8-inta-m-phenylene prostaglandin $I_2$ derivatives represented by the following general formula (I)

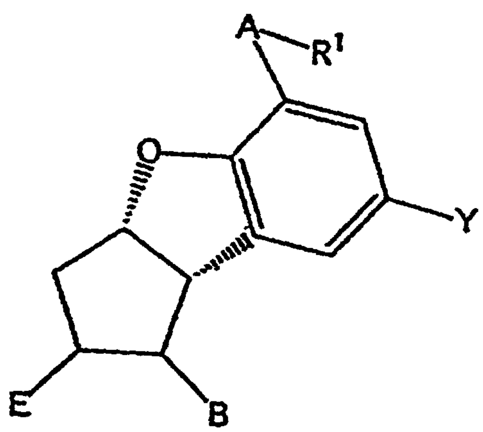

(I)

[in the formula, $R^1$ denotes

(A) $COOR^2$, wherein $R^2$ represents

1) hydrogen, or a pharmaceutically acceptable cation,
2) a linear alkyl group with a carbon number of 1 to 12, or a branched alkyl group with a carbon number of 3 to 14,
3) $-Z-R^3$,
wherein Z denotes a valence bond, or a linear or branched alkylene group represented by $C_tH_{2t}$ in which t represents an integer of 1 to 6, $R^3$ denotes a cycloalkyl group with a carbon number of 3 to 12 or a substituted cycloalkyl group with a carbon number of 3 to 12 substituted with one to three $R^4$ groups, and $R^4$ denotes hydrogen or an alkyl group with a carbon number of 1 to 5,
4) $-(CH_2CH_2O)_nCH_3$,
wherein n is an integer of 1 to 5,
5) $-Z-AR^1$
wherein Z is the same as defined above, and $Ar^1$ denotes phenyl, α-naphthyl, β-naphthyl, 2-pyridyl, 3-pyridyl, 4-pyridyl, α-furyl, β-furyl, α-thienyl, β-thienyl, or a substituted phenyl group (wherein the substituent is at least one chlorine, bromine, fluorine, iodine, trifluoromethyl group, alkyl group with a carbon number of 1 to 4, or nitro, cyano, methoxy, phenyl, phenoxy, p-acetamidebenzamide, -CH=N-NH-C(=O) $-NH_2$, -NH-C(=O)-Ph, -NH-C(=O)-$CH_3$ or -NH-C(=O)-$NH_2$ group),
6) $-C_tH_{2t}COOR^4$,
wherein $C_tH_{2t}$ and $R^4$ are the same as defined above,
7) $-C_tH_{2t}N(R^4)_2$,
wherein $C_tH_{2t}$ and $R^4$ are the same as defined above,
8) $-CH(R^5)-C(=O)-R^6$,
wherein $R^5$ is hydrogen or a benzoyl group, $R^6$ is a phenyl, p-bromophenyl, p-chlorophenyl, p-biphenyl, p-nitrophenyl, p-benzamidephenyl or 2-naphthyl group,
9) $-C_pH_{2p}-W-R^7$,
wherein W represents -CH=CH-, $-CH=CR^7-$ or -C≡C- group, $R^7$ represents hydrogen or a linear or branched alkyl or aralkyl group with a carbon number of 1 to 30, and p denotes an integer of 1 to 5, or
10) $-CH(CH_2OR^8)_2$,
wherein $R^8$ denotes an alkyl or acyl group with a carbon number of 1 to 30,

(B) $-CH_2OH$,
(C) $-C(=O)N(R^9)_2$,
wherein $R^9$ is hydrogen, a linear alkyl group with a carbon number of 1 to 12, a branched alkyl group with a carbon number of 3 to 12, a cycloalkyl group with a carbon number of 3 to 12, a cycloalkylalkylene group with a carbon number of 4 to 13, a phenyl or a substituted phenyl group (the substituent is the same as in (A) 5)), an aralkyl group with a carbon number of 7 to 12 or $-SO_2R^{10}$, wherein $R^{10}$ denotes an alkyl group with a carbon number of 1 to 10, a cycloalkyl group with a carbon number of 3 to 12, or a phenyl or substituted phenyl group (the substituent is the same as in (A) 5)) or an aralkyl group with a carbon number of 7 to 12, in which the two $R^9$ groups may be the same or different, but one of the $R^9$ group is not $-SO_2R^{10}$ group when the other $R^9$ group represents $-SO_2R^{10}$ group, or
(D) $-CH_2OTHP$ (THP represents tetrahydropyranyl group),
wherein A is

1) $-(CH_2)_m-$
2) $-CH=CH-CH_2-$,
3) $-CH_2-CH=CH-$,
4) $-CH_2-O-CH_2-$,
5) -CH=CH-,
6) $-O-CH_2-$, or
7) -C≡C-,

wherein m denotes an integer of 1 to 3, and
Y represents hydrogen, an alkyl group with a carbon number of 1 to 4, chlorine, bromine, or fluorine, or a formyl, methoxy or nitro group,
B is $-X-C(R^{11})(R^{12})OR^{13}$,

wherein $R^{11}$ represents hydrogen or an alkyl group with a carbon number of 1 to 4, $R^{13}$ represents hydrogen, an acyl group with a carbon number of 1 to 14, an aroyl group with a carbon number of 6 to 15, or a tetrahydropyranyl, tetrahydrofuranyl, 1-ethoxyethyl or t-butyl group, X is

1) $-CH_2-CH_2-$,
2) -CH=CH- or
3) -C≡C-,

$R^{12}$ is

1) a linear alkyl group with a carbon number of 1 to 12, a branched alkyl group with a carbon number of 3 to 14, or
2) $-Z-Ar^2$
wherein Z is the same as defined above, and $Ar^2$ represents a phenyl, α-naphthyl or β-naphthyl group, or a phenyl group substituted with at least one chlorine, bromine, fluorine, iodine, trifluoromethyl group, alkyl group with a carbon number of 1 to 4, nitro, cyano, methoxy, phenyl or phenoxy group, or
3) $-C_tH_{2t}OR^{14}$,
wherein $C_tH_{2t}$ is the same as defined above, and $R^{14}$ represents a linear alkyl group with a carbon number of 1 to 6, a branched alkyl group with a carbon number of 3 to 6, a phenyl group, a phenyl group substituted with at least one chlorine, bromine, fluorine, iodine, trifluoromethyl group, alkyl group with a carbon number of 1 to 4, nitro, cyano, methoxy, phenyl or phenoxy groups, or a cyclopentyl or cyclohexyl group, or a cyclopentyl or cyclohexyl group substituted with one to four linear alkyl groups with a carbon number of 1 to 4, or
4) $-Z-R^3$,
wherein Z and $R^3$ are the same as defined above, or
5) $C_tH_{2t}-CH=C(R^{15})R^{16}$,
wherein $C_tH_{2t}$ is the same as defined above, $R^{15}$ and $R^{16}$ represent hydrogen, a methyl, ethyl, propyl or butyl group, or
6) $-C_uH_{2u}-C{\equiv}C-R^{17}$,
wherein u is an integer of 1 to 7, $C_uH_{2u}$ represents a linear or branched alkylene group, and $R^{17}$ represents a linear alkyl group with a carbon number of 1 to 6, and
E denotes hydrogen or $-OR^{18}$,
wherein $R^{18}$ denotes an acyl group with a carbon number of 1 to 12, an aroyl group with a carbon number of 7 to 15, or $R^2$ ($R^2$ is the same as defined above),
wherein the general formula represents a d-isomer, a 1-isomer or a dl-isomer],

or a pharmaceutically acceptable salt thereof.

Examples of the pharmaceutically acceptable salts include an alkali metal salt such as a sodium salt and potassium salt, an alkali earth metal salt such as a calcium salt and magnesium salt, an amine salt such as methylamine salt, dimethylamine salt, trimethylamine salt, methypiperidine salt, ethanolamine salt, diethanolamine salt, triethanolamine salt, and lysin salt, an ammonium salt or a basic amino acid salt.

The prostaglandin EP4 receptor ligands according to the present invention is very useful as a pharmaceutical tool for making physiological functions related to prostaglandin EP4 receptor clear, or as a medicine for use in prevention or treatment of prostaglandin EP4 receptor-mediated diseases. In other words, since the prostaglandin EP4 receptor has been already known to be distributed in such organs as the heart, kidney, liver, intestine, lung and bone, the compounds that bind to prostaglandin EP4 receptor have a possibility to serve as preventives or remedies for the diseases of these organs. For example, since it is made clear that the compound according to the present invention has a suppressive property of collagen production in Example 2, it may be effective for prevention or therapy of the diseases caused by collagen production. The compound may be also used for treatment of prostaglandin EP4 receptor-mediated diseases.

While one or several kinds of the prostaglandin $I_2$ derivatives or salts thereof may be directly administered when the prostaglandin $I_2$ derivative according to the present invention is clinically used as a medicine, it may be appropriately mixed with an additives such as an excipient, a stabilizer, a preservative, a buffer, a dissolution aid, an emulsifier, a dilluent, and an isotonic agent. The compounds are systemically or locally administered in the form of oral or non-oral preparations. The compound is administered in a formulation such as tablets, pills, solutions, capsules, granules and powders for oral administration, or injections, liquid for external application, ointments, gel, suppositories and pessaries for non-oral administration. These preparations may be manufactured by the conventional formulation technologies. Although the dosage of the agents differ depending on the conditions, age ,body weight and type of the administration in patients, it is usually 0.0001 mg to 1 g, preferably 0.001 mg to 500 mg, per one adult a day, which may

be administered at once or in several portions.

[Examples]

The present invention will be described in more detail with reference to the examples.

Example 1

Receptor binding study using $PGE_2$ receptor subtype expression cells:

The compound represented by the following formula (II) among the compounds represented by the general formula (I) strongly binds to the EP4 receptor as a subtype of the $PGE_2$ receptor. The binding activity was confirmed in comparison with $PGE_2$ by an experiment using the $PGE_2$ receptor subtype expression cells.

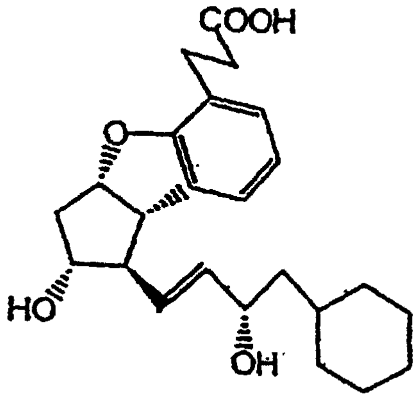

(II)

The experiment was carried out according to the previous report [Breyer, R. M. et al., J. Biol. Chem. 269, 6163-6169 (1994)], wherein the human EP4 receptor subtype was transiently expressed using COS-7 cells. These cells were prepared as membrane samples. The membrane fraction prepared (10 µg/tube) was incubated with reaction solution containing $^3H$-$PGE_2$ at 30°C for 1 hour. The reaction was terminated with ice-cold buffer [10mM MES (pH6.0), 10mM $MgCl_2$, 1mM EDTA], and the bound $^3H$-$PGE_2$ was trapped on a glass filter (GF/C) by vacuum filtration under a reduced pressure, followed by counting the radioactivity with a liquid scintillator.

The Kd value and Bmax were determined from a Scatchard plot according to the conventional method. The non-specific binding was determined by the amount of binding in the presence of an excess amount (5 µM) on the non-labeled $PGE_2$. Inhibitory action of the $^3H$-$PGE_2$ binding by the compound to be tested was determined at a concentration of $^3H$-$PGE_2$ of 5 nM by adding various concentrations of the compound to be tested.

The dissociation constant Ki (nM) of the compound to be tested was determined by the following equation:

$$Ki = IC_{50}/(1 + ([C])/kd)$$

The result is shown in Table 1.

[TABLE 1]

| | DISSOCIATION CONSTANT Ki (nM) EP4 |
|---|---|
| COMPOUND (II) | 19 |

Example 2

Effects on collagen production in human normal lung fibroblast cells:

The suppression action of the compound represented by the formula (II) on the collagen production from the fibroblast cells was confirmed by the following test method. After the human normal lung fibroblast cells were cultured for 48 hours under the condition of confluence (Eagle MEM culture medium with 0.45% FBS), the cells were added(1 μM) the compound represented by the formula (II) and transforming growth factor β1 (TGF-β1) at a concentration of 5 ng/ml, then incubated for 24 hours. The supernatant from the cell culture medium was concentrated by freeze-drying, and the amount of collagen (salt-soluble collagen) was qualitatively assayed using Siricol Collagen Assay Kit (made by Biocolor Co.). The amounts of collagen in the supernatant were as follows:

| | |
|---|---|
| Reference: | 1.1 μg/100,000 cells |
| Compound (II): | 0.5 μg/100,000 cells |

It was confirmed that production of collagen by TGF-β1 was suppressed by the compound according to the present invention.

Industrial Applicability

It was made clear that the prostaglandin $I_2$ derivatives as the compounds according to the present invention strongly bind to the prostaglandin EP4 receptor. In other words, the compound according to the present invention is useful as a pharmaceutical tool for making physiological actions related to prostaglandin EP4 receptor clear, or as a medicine for use in prevention/treatment of prostaglandin EP4 receptor-mediated diseases.

**Claims**

**1.** A prostaglandin EP4 receptor ligand comprising a prostaglandin $I_2$ derivative as an effective ingredient.

**2.** A prostaglandin EP4 receptor ligand according to Claim 1, wherein the prostaglandin $I_2$ derivative comprises a methaphenylene, carbacyclin or isocarbacyclin type prostaglandin $I_2$ derivative.

**3.** A prostaglandin EP4 receptor ligand according to Claim 2, wherein the methaphenylene type prostaglandin $I_2$ derivative is a 4,8-inta-m-phenylene prostaglandin $I_2$ derivative represented by the following general formula (I)

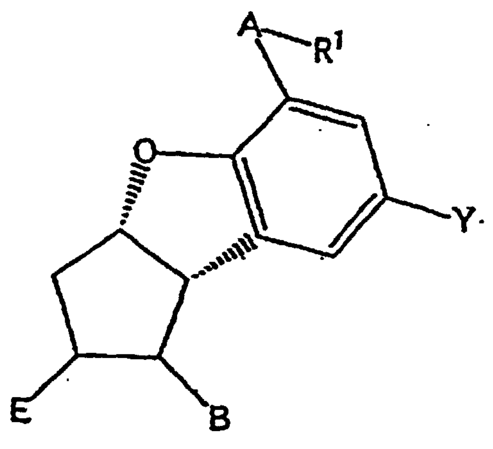

(I)

[in the formula, $R^1$ denotes

(A) $COOR^2$, wherein $R^2$ represents

1) hydrogen, or a pharmaceutically acceptable cation,
2) a linear alkyl group with a carbon number of 1 to 12, or a branched alkyl group with a carbon number of

3 to 14,

3) $-Z-R^3$,

wherein Z denotes a valence bond, or a linear or branched alkylene group represented by $C_tH_{2t}$ in which t represents an integer of 1 to 6, $R^3$ denotes a cycloalkyl group with a carbon number of 3 to 12 or a substituted cycloalkyl group with a carbon number of 3 to 12 substituted with one to three $R^4$ groups, and $R^4$ denotes hydrogen or an alkyl group with a carbon number of 1 to 5,

4) $-(CH_2CH_2O)_nCH_3$,

wherein n is an integer of 1 to 5,

5) $-Z-Ar^1$

wherein Z is the same as defined above, and $Ar^1$ denotes phenyl, α-naphthyl, β-naphthyl, 2-pyridyl, 3-pyridyl, 4-pyridyl, α-furyl, β-furyl, α-thienyl, β-thienyl, or a substituted phenyl group (wherein the substituent is at least one chlorine, bromine, fluorine, iodine, trifluoromethyl group, alkyl group with a carbon number of 1 to 4, or nitro, cyano, methoxy, phenyl, phenoxy, p-acetamidebenzamide, $-CH=N-NH-C(=O)-NH_2$, -NH-C(=O)-Ph, $-NH-C(=O)-CH_3$ or $-NH-C(=O)-NH_2$ group),

6) $-C_tH_{2t}COOR^4$,

wherein $C_tH_{2t}$ and $R^4$ are the same as defined above,

7) $-C_tH_{2t}N(R^4)_2$,

wherein $C_tH_{2t}$ and $R^4$ are the same as defined above,

8) $-CH(R^5)-C(C=O)-R^6$,

wherein $R^5$ is hydrogen or a benzoyl group, $R^6$ is a phenyl, p-bromophenyl, p-chlorophenyl, p-biphenyl, p-nitrophenyl, p-benzamidephenyl or 2-naphthyl group,

9) $-C_pH_{2p}-W-R^7$,

wherein W represents -CH=CH-, $-CH=CR^7-$ or -C≡C- group, $R^7$ represents hydrogen or a linear or branched alkyl or aralkyl group with a carbon number of 1 to 30, and p denotes an integer of 1 to 5, or

10) $-CH(CH_2OR^8)_2$,

wherein $R^8$ denotes an alkyl or acyl group with a carbon number of 1 to 30,

(B) $-CH_2OH$,

(C) $-C(=O)N(R^9)_2$,

wherein $R^9$ is hydrogen, a linear alkyl group with a carbon number of 1 to 12, a branched alkyl group with a carbon number of 3 to 12, a cycloalkyl group with a carbon number of 3 to 12, a cycloalkylalkylene group with a carbon number of 4 to 13, a phenyl or a substituted phenyl group (the substituent is the same as in (A) 5)), an aralkyl group with a carbon number of 7 to 12 or $-SO_2R^{10}$, wherein $R^{10}$ denotes an alkyl group with a carbon number of 1 to 10, a cycloalkyl group with a carbon number of 3 to 12, or a phenyl or substituted phenyl group (the substituent is the same as in (A) 5)) or an aralkyl group with a carbon number of 7 to 12, in which the two $R^9$ groups may be the same or different, but one of the $R^9$ group is not $-SO_2R^{10}$ group when the other $R^9$ group represents $-SO_2R^{10}$ group, or

(D) $-CH_2OTHP$ (THP represents tetrahydropyranyl group),

wherein A is

1) $-(CH_2)_m-$

2) $-CH=CH-CH_2-$,

3) $-CH_2-CH=CH-$,

4) $-CH_2-O-CH_2-$,

5) -CH=CH-,

6) $-O-CH_2-$, or

7) -C≡C-,

wherein m denotes an integer of 1 to 3, and

Y represents hydrogen, an alkyl group with a carbon number of 1 to 4, chlorine, bromine, or fluorine, or a formyl, methoxy or nitro group,

B is $-X-C(R^{11})(R^{12})OR^{13}$,

wherein $R^{11}$ represents hydrogen or an alkyl group with a carbon number of 1 to 4, $R^{13}$ represents hydrogen, an acyl group with a carbon number of 1 to 14, an aroyl group with a carbon number of 6 to 15, or a tetrahydropyranyl, tetrahydrofuranyl, 1-ethoxyethyl or t-butyl group, X is

1) $-CH_2-CH_2-$,

2) -CH=CH- or

3) -C≡C-,

$R^{12}$ is

1) a linear alkyl group with a carbon number of 1 to 12, a branched alkyl group with a carbon number of 3 to 14, or
2) -Z-$Ar^2$
wherein Z is the same as defined above, and $Ar^2$ represents a phenyl, α-naphthyl or β-naphthyl group, or a phenyl group substituted with at least one chlorine, bromine, fluorine, iodine, trifluoromethyl group, alkyl group with a carbon number of 1 to 4, nitro, cyano, methoxy, phenyl or phenoxy group, or
3) $-C_tH_{2t}OR^{14}$,
wherein $C_tH_{2t}$ is the same as defined above, and $R^{14}$ represents a linear alkyl group with a carbon number of 1 to 6, a branched alkyl group with a carbon number of 3 to 6, a phenyl group, a phenyl group substituted with at least one chlorine, bromine, fluorine, iodine, trifluoromethyl group, alkyl group with a carbon number of 1 to 4, nitro, cyano, methoxy, phenyl or phenoxy group, or a cyclopentyl or cyclohexyl group, or a cyclopentyl or cyclohexyl group substituted with one to four linear alkyl groups with a carbon number of 1 to 4, or
4) -Z-$R^3$,
wherein Z and $R^3$ are the same as defined above, or
5) $C_tH_{2t}$-CH=C($R^{15}$)$R^{16}$,
wherein $C_tH_{2t}$ is the same as defined above, $R^{15}$ and $R^{16}$ represent hydrogen, a methyl, ethyl, propyl or butyl group, or
6) $-C_uH_{2u}$-C≡C-$R^{17}$,

wherein u is an integer of 1 to 7, $C_uH_{2u}$ represents a linear or branched alkylene group, and $R^{17}$ represents a linear alkyl group with a carbon number of 1 to 6, and
E denotes hydrogen or -$OR^{18}$,
wherein $R^{18}$ denotes an acyl group with a carbon number of 1 to 12, an aroyl group with a carbon number of 7 to 15, or $R^2$ ($R^2$ is the same as defined above),
wherein the general formula represents a d-isomer, a l-isomer or a dl-isomer],

or a pharmaceutically acceptable salt thereof.

**4.** A prostaglandin EP4 receptor ligand according to Claim 3, wherein A is $-(CH_2)_2-$, -CH=CH- or $-O-CH_2-$.

**5.** A collagen production suppressing agent comprising the prostaglandin EP4 receptor ligand according to Claims 1 to 4 as an effective ingredient.

**6.** A method for treating the prostaglandin EP4 receptor-mediated diseases, wherein an effective dosage of a prostaglandin $I_2$ derivative is administered.

**7.** A treatment method according to Claim 6, wherein the prostaglandin $I_2$ derivative is a methaphenylene, carbacyclin or isocarbacyclin type prostaglandin $I_2$ derivative.

**8.** A treatment method according to Claim 7, wherein the methaphenylene type prostaglandin $I_2$ derivative is a 4,8-inta-m-phenylene prostaglandin $I_2$ derivative represented by the following general formula (I)

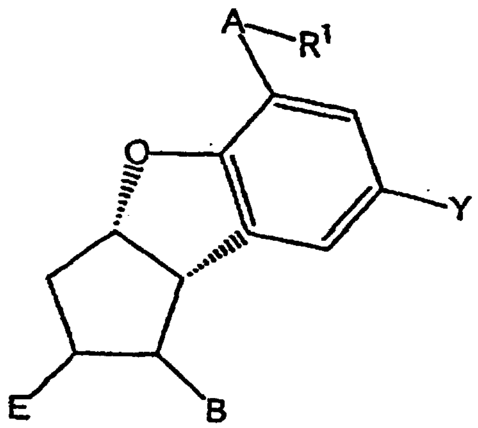

(I)

[in the formula, $R^1$ denotes

(A) $COOR^2$, wherein $R^2$ represents

1) hydrogen, or a pharmaceutically acceptable cation,
2) a linear alkyl group with a carbon number of 1 to 12, or a branched alkyl group with a carbon number of 3 to 14,
3) $-Z-R^3$,
wherein Z denotes a valence bond, or a linear or branched alkylene group represented by $C_tH_{2t}$ in which t represents an integer of 1 to 6, $R^3$ denotes a cycloalkyl group with a carbon number of 3 to 12 or a substituted cycloalkyl group with a carbon number of 3 to 12 substituted with one to three $R^4$ groups, and $R^4$ denotes hydrogen or an alkyl group with a carbon number of 1 to 5,
4) $-(CH_2CH_2O)_nCH_3$,
wherein n is an integer of 1 to 5,
5) $-Z-Ar^1$
wherein Z is the same as defined above, and $Ar^1$ denotes phenyl, α-naphthyl, β-naphthyl, 2-pyridyl, 3-pyridyl, 4-pyridyl, α-furyl, β-furyl, α-thienyl, β-thienyl, or a substituted phenyl group (wherein the substituent is at least one chlorine, bromine, fluorine, iodine, trifluoromethyl group, alkyl group with a carbon number of 1 to 4, or nitro, cyano, methoxy, phenyl, phenoxy, p-acetamide, benzamide, -CH=N-NH-C(=O)-, $-NH_2$, -NH-C(=O)-Ph, $-NH-C(=O)-CH_3$ or $-NH-C(=O)-NH_2$ group),
6) $-C_tH_{2t}COOR^4$,
wherein $C_tH_{2t}$ and $R^4$ are the same as defined above,
7) $-C_tH_{2t}N(R^4)_2$,
wherein $C_tH_{2t}$ and $R^4$ are the same as defined above,
8) $-CH(R^5)-C(=O)-R^6$,
wherein $R^5$ is hydrogen or a benzoyl group, $R^6$ is a phenyl, p-bromophenyl, p-chlorophenyl, p-biphenyl, p-nitrophenyl, p-benzamidephenyl or 2-naphthyl group,
9) $-C_pH_{2p}-W-R^7$,
wherein W represents -CH=CH-, $-CH=CR^7-$ or -C≡C- group, $R^7$ represents hydrogen or a linear or branched alkyl or aralkyl group with a carbon number of 1 to 30, and p denotes an integer of 1 to 5, or
10) $-CH(CH_2OR^8)_2$,
wherein $R^8$ denotes an alkyl or acyl group with a carbon number of 1 to 30,

(B) $-CH_2OH$,
(C) $-C(=O)N(R^9)_2$,
wherein $R^9$ is hydrogen, a linear alkyl group with a carbon number of 1 to 12, a branched alkyl group with a carbon number of 3 to 12, a cycloalkyl group with a carbon number of 3 to 12, a cycloalkylalkylene group with a carbon number of 4 to 13, a phenyl or a substituted phenyl group (the substituent is the same as in (A) 5)), an aralkyl group with a carbon number of 7 to 12 or $-SO_2R^{10}$, wherein $R^{10}$ denotes an alkyl group with a carbon number of 1 to 10, a cycloalkyl group with a carbon number of 3 to 12, or a phenyl or substituted phenyl

group (the substituent is the same as in (A) 5)) or an aralkyl group with a carbon number of 7 to 12, in which the two $R^9$ groups may be the same or different, but one of the $R^9$ group is not $-SO_2R^{10}$ group when the other $R^9$ group represents $-SO_2R^{10}$ group, or

(D) -CH2OTHP (THP represents tetrahydropyranyl group),

wherein A is

1) $-(CH_2)_m-$
2) $-CH=CH-CH_2-$,
3) $-CH_2-CH=CH-$,
4) $-CH_2-O-CH_2-$,
5) -CH=CH-,
6) $-O-CH_2-$, or
7) -C≡C-,

wherein m denotes an integer of 1 to 3, and

Y represents hydrogen, an alkyl group with a carbon number of 1 to 4, chlorine, bromine, or fluorine, or a formyl, methoxy or nitro group,

B is $-X-C(R^{11})(R^{12})OR^{13}$,

wherein $R^{11}$ represents hydrogen or an alkyl group with a carbon number of 1 to 4, $R^{13}$ represents hydrogen, an acyl group with a carbon number of 1 to 14, an aroyl group with a carbon number of 6 to 15, or a tetrahydropyranyl, tetrahydrofuranyl, 1-ethoxyethyl or t-butyl group, X is

1) $-CH_2-CH_2-$,
2) -CH=CH- or
3) -C≡C-,

$R^{12}$ is

1) a linear alkyl group with a carbon number of 1 to 12, a branched alkyl group with a carbon number of 3 to 14, or
2) $-Z-Ar^2$
wherein Z is the same as defined above, and $Ar^2$ represents a phenyl, α-naphthyl or β-naphthyl group, or a phenyl group substituted with at least one chlorine, bromine, fluorine, iodine, trifluoromethyl group, alkyl group with a carbon number of 1 to 4, nitro, cyano, methoxy, phenyl or phenoxy group, or
3) $-C_tH_{2t}OR^{14}$,
wherein $C_tH_{2t}$ is the same as defined above, and $R^{14}$ represents a linear alkyl group with a carbon number of 1 to 6, a branched alkyl group with a carbon number of 3 to 6, a phenyl group, or phenyl group substituted with at least one chlorine, bromine, fluorine, iodine, trifluoromethyl group, alkyl group with a carbon number of 1 to 4, nitro, cyano, methoxy, phenyl or phenoxy group, or a cyclopentyl or cyclohexyl group, or a cyclopentyl or cyclohexyl group substituted with one to four linear alkyl groups with a carbon number of 1 to 4, or
4) $-Z-R^3$,
wherein Z and $R^3$ are the same as defined above, or
5) $C_tH_{2t}-CH=C(R^{15})R^{16}$,
wherein $C_tH_{2t}$ is the same as defined above, $R^{15}$ and $R^{16}$ represent hydrogen, a methyl, ethyl, propyl or butyl group, or
6) $-C_uH_{2u}-C≡C-R^{17}$,

wherein u is an integer of 1 to 7, $C_uH_{2u}$ represents a linear or branched alkylene group, and $R^{17}$ represents a linear alkyl group with a carbon number of 1 to 6, and

E denotes hydrogen or $-OR^{18}$,

wherein $R^{18}$ denotes an acyl group with a carbon number of 1 to 12, an aroyl group with a carbon number of 7 to 15, or $R^2$ ($R^2$ is the same as defined above),

wherein the general formula represents a d-isomer, a l-isomer or a dl-isomer],

or a pharmaceutically acceptable salt thereof.

**9.** A treatment method according to Claim 8, wherein A is $-(CH_2)_2-$, -CH=CH- or $-O-CH_2-$.

## INTERNATIONAL SEARCH REPORT

International application No.
PCT/JP00/01556

**A. CLASSIFICATION OF SUBJECT MATTER**
Int.Cl⁷ A61K45/00, A61K31/557, A61P43/00, A61P13/12

According to International Patent Classification (IPC) or to both national classification and IPC

**B. FIELDS SEARCHED**

Minimum documentation searched (classification system followed by classification symbols)
Int.Cl⁷ A61K45/00, A61K31/557, A61P43/00, A61P13/12

Documentation searched other than minimum documentation to the extent that such documents are included in the fields searched

| | | | |
|---|---|---|---|
| Jitsuyo Shinan Koho | 1940-1992 | Toroku Jitsuyo Shinan Koho | 1994-1996 |
| Kokai Jitsuyo Shinan Koho | 1971-1992 | Jitsuyo Shinan Toroku Koho | 1996-2000 |

Electronic data base consulted during the international search (name of data base and, where practicable, search terms used)
CA(STN),MEDLINE(STN),EMBASE(STN),BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| X<br>A | YU Hisahiro, "Prostacyclin Release by Rat Cardiac Fibroblasts Inhibition of Collagen Expression", Hypertension, Vol.30, No.5 (1997), P.1047-1053, Full text, especially, P.1048, left column, lines 5~9; P.1050, right column, line 7 to P.1051, left column, line 7; P.1052, right column, lines 8~36 | 5<br>1-4 |
| X<br>A | GALLAGHER Ann, "Bradykinin-Induced Reductions in Collagen Gene Expression Involve Prostacyclin", Hypertension, Vol.32, No.1 (1998), P.84-88, Full text, especially, lines 9~15 in ABSTRACT; P.84, right column, lines 1~4; RESULTS | 5<br>1-4 |
| A | KIRIYAMA Michitaka, "Ligand binding specificities of the eight types and subtypes of the mouse prostanoid receptors expressed in Chinese hamster ovary cells", Br. J. Pharmacol., Vol.122, No.2 (1997), P.217-224 | 1-5 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 June, 2000 (05.06.00) | 13 June, 2000 (13.06.00) |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP00/01556

**Box I Observations where certain claims were found unsearchable (Continuation of item 1 of first sheet)**

This international search report has not been established in respect of certain claims under Article 17(2)(a) for the following reasons:

1. ☒ Claims Nos.: 6-9
because they relate to subject matter not required to be searched by this Authority, namely:

The subject matter of claims 6-9 relates to a method for treatment of the human body by therapy, which does not require an international search report by the International Search Authority in accordance with PCT Article 17(2)(a)(i)and Rule 39.1(iv).

2. ☒ Claims Nos.: 1
because they relate to parts of the international application that do not comply with the prescribed requirements to such an extent that no meaningful international search can be carried out, specifically:

Although the term "Prostaglandin I2 derivative" involves various compounds, use of the word "derivative" makes it unclear what particular constituents are involved therein or not. Therefore, the International Search cannot be fully practiced on the corresponding claims.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)